# EUROPÄISCHE PATENTSCHRIFT

(11) **EP 2 777 801 B1**
(45) Veröffentlichungstag und Bekanntmachung des Hinweises auf die Patenterteilung: **28.08.2019**
(21) Anmeldenummer: 13168103.3
(22) Anmeldetag: 16.05.2013
(51) Int. Cl.: B01D 63/02, A61M 1/16, A61M 1/36, B01D 19/00

(54) **Vorrichtung zur CO2-Eliminierung von Patientenblut**
Device for eliminating CO2 from patient blood
Dispositif d'élimination du CO2 du sang d'un patient

(30) Priorität: 15.03.2013 US 201361802335 P
(43) Veröffentlichungstag der Anmeldung: 17.09.2014
(73) Patentinhaber: Maquet Cardiopulmonary AG, 76437 Rastatt (DE)
(72) Erfinder: Kober, Rudolf, 76534 Baden-Baden (DE); Haag, Ulrich, 72406 Bisingen (DE); Thölke, Ralf, 15732 Eichwalde (DE); Nakel, Mathias, 72393 Burladingen (DE); Möllenberg, Oliver, 83607 Holzkirchen (DE)
(74) Vertreter: Kohler Schmid Möbus Patentanwälte

(56) Entgegenhaltungen:
- EP-A1- 0 306 613
- EP-A2- 0 521 495
- EP-A2- 1 810 704
- EP-A2- 1 864 709
- DE-A1-102009 008 601
- DE-U1- 20 011 060
- JP-A- 2000 084 369

## Beschreibung

Die Erfindung betrifft eine Vorrichtung zur mindestens teilweisen Eliminierung von CO₂ aus Patientenblut im extrakorporalen Kreislauf mit einem CO₂-Eliminator, der ein Gehäuse mit einem Bluteinlass und einem Blutauslass sowie einem Gaseinlass und einem Gasauslass aufweist, wobei im Gehäuse eine sowohl vom Gas als auch Blut durchströmbare Hohlfaseranordnung mit einer aktiven Faserlänge vorgesehen ist.

Die Erkrankung COPD (Chronic Obstructive Pulmonary Desease) führt zu einer Behinderung der Ausatmung und in Folge zu chronischer respiratorischer Insuffizienz. Die Symptome sind Atemnot und oft erhöhte pCO₂-Werte. Bei dauerhaft erhöhten pCO₂-Werten (Hyperkapnie, Ventilationsinsuffizienz) ist Hilfe über eine Beatmung möglich, die jedoch wegen des angewandten Überdrucks und der forcierten Füllung mit möglicher Überdehnung der Lunge zu Lungenschäden führen kann.

Therapieverfahren wie die pumpenlose extrakorporale Lungenunterstützung (Interventional Lung Assist (iLA)) oder extracorporal membrane oxygenation (ECMO)) können in dieser Situation den Atemantrieb und die Atemarbeit reduzieren, sodass die Atemnot verschwindet und eine Erholung des Patienten möglich wird.

Beispielsweise aus der DE 200 11 060 U1 ist eine Vorrichtung zur Unterstützung der Lungenfunktion eines Patienten bekannt. Diese Vorrichtung ist auf einen sehr geringen Strömungswiderstand der Blutseite ausgelegt, sodass auf eine antreibende Pumpe bei arterio-venösem (A-V)-Anschluss verzichtet werden kann. Allerdings benötigt diese Vorrichtung einen Mindestblutfluss von 0,5 l pro Minute, um effektiv arbeiten zu können, und benötigt bei passivem AV-Betrieb verhältnismäßig großlumige Kanülen. Damit ist diese Vorrichtung nur bei einer großen Invasivität effektiv. Aufgrund ihrer Bauart ist diese Vorrichtung vorrangig als Oxygenator, bzw. bei verhältnismäßig hohem Blutfluss auch zur CO₂-Eliminierung (Massentransfer) geeignet.

Das bekannte System hat den Nachteil, dass der Blutfluss abhängig von der AV-Druckdifferenz und dem blutseitigen Strömungswiderstand ist. Für eine gute Effizienz sind daher zwei getrennte Zugänge für die Zu- oder Abfuhr des Patientenblutes mit großlumigen Kanülen/Kathetern und Schlauchsystemen und entsprechenden Gefäßzugängen erforderlich, was eine hohe Invasivität bedingt. Dies bedeutet höheres Primingvolumen und stärkere Beeinträchtigung des Patienten durch die zusätzlichen größeren Wundflächen und eine Anwendung nur in der Intensivmedizin, wo solche Gefäßzugänge gelegt werden können. Zusätzlich muss der extrakorporale Kreislauf wegen des hohen Blutflusses intensiv überwacht werden, da bei einer Leckage oder Diskonnektierung innerhalb weniger Sekunden für den Patienten Lebensgefahr besteht.

Die gleichen Aussagen gelten auch für die ECMO-Anwendungen von Oxygenatoren. Auch hier werden zwei getrennte Zugänge für die Zu- oder Abfuhr des Patientenblutes mit großlumigen Kanülen/Kathetern und Schlauchsystemen und entsprechende Gefäßzugänge benötigt, was eine hohe Invasivität bedingt. Wegen der hohen Invasivität von ECMO-Anwendungen werden diese oft als letzte lebenserhaltende Maßnahme angesehen und angewendet, was zu hoher Mortalität und deshalb zu einer eher schlechten Akzeptanz dieser an sich guten Behandlungsmethode führt.

Aus der EP 1 522 323 A1 ist eine Vorrichtung bekannt, die aus einem in einem Gehäuse integrierten Oxygenator mit nachgeschaltetem Dialysator besteht. In der EP 1 524 000 A8 wird die Anwendung einer solchen Vorrichtung in einem System zur kombinierten Hämodialyse und CO₂-Eliminierung offenbart.

Aus der EP 0 306 613 A1 und der JP 2000 084369 A sind Vorrichtungen bekannt, die in einem Innenraum Fasern aufweisen, wobei der Innenraum sowohl von Gas als auch Blut durchströmbar ist, um einen Gasaustausch zu bewirken.

Die DE 10 2009 008 601 A1 offenbart eine Vorrichtung zur Behandlung einer biologischen Flüssigkeit, umfassend mindestens drei Kammern, wobei eine erste Kammer, die zur Aufnahme der biologischen Flüssigkeit bestimmt ist, und eine zweite Kammer, die zur Aufnahme eines Gases bestimmt ist, durch mindestens eine gasdurchlässige und flüssigkeitsundurchlässige Membran voneinander getrennt sind.

Die EP 1 864 709 A2 offenbart eine Vorrichtung, die in ihrem Innenraum einen Stapel von Membranmatten aufweist.

Aus der DE 200 11 060 U1 ist eine Vorrichtung zur Unterstützung der Lungenfunktion eines Patienten bekannt.

Die EP 1 810 704 A2 offenbart einen Oxygenator mit vorgeschaltetem Wärmetauscher und nachgeschaltetem Filter.

Die EP 0 521 495 A2 offenbart eine Vorrichtung zur Stoffübertragung von einem Gas auf eine Flüssigkeit oder umgekehrt mittels geradlinig ausgebildeten Hohlfäden, bei welcher das Gas durch den Innenhohlraum der Hohlfäden strömt und die Endbereiche der Hohlfäden in einem Vergussmassekörper eingebettet sind.

Aufgabe der vorliegenden Erfindung ist es, eine Vorrichtung für die mindestens teilweise Eliminierung von CO₂ bei Hyperkapnie bereitzustellen, die hohe Effektivität bei geringer Invasivität bietet.

Gelöst wird diese Aufgabe erfindungsgemäß durch eine Vorrichtung zur zumindest teilweisen CO₂-Eliminierung von Patientenblut im extrakorporalen Kreislauf mit den Merkmalen des Anspruchs 1.

Dabei ist die aktive Faserlänge vorzugsweise eine Dimension des Querschnitts der Hohlfaseranordnung, der durch zu behandelndes Patientenblut durchströmbar ist. Die Wegstrecke der Blutpassage ist dabei der kürzeste Weg, den das zu behandelnde Blut durch die Hohlfaseranordnung nehmen kann. Insbesondere kann die minimale Wegstrecke der Blutpassage einer (weiteren) Dimension der Hohlfaseranordnung entsprechen. Die aktive Faserlänge und die minimale Wegstrecke können dabei einen Winkel von 90° zueinander aufweisen. Mit der erfindungsgemäßen Vorrichtung kann der CO₂-Gehalt im Blut eines Patienten verringert werden.

Die Hohlfasern sind dabei in Matten angeordnet, wobei die Fasern in den Matten parallel ausgerichtet sind. Die Matten sind in der Hohlfaseranordnung gestapelt, sodass die Fasern in parallelen benachbarten Matten in etwa rechtwinklig zu einander stehen. Die Wegstrecke bzw. die Blutflussrichtung ist im Wesentlichen rechtwinklig zur Faserorientierung. Die Hohlfasern können entweder vom mikroporösen oder vom diffusiven Typ sein. Beispiele für den mikroporösen bzw. diffusiven Typ sind die mikroporöse PP-Hohlfaser Oxyphan bzw. die diffusive PMP-Hohlfaser Oxyplus der Firma Membrana.

Die Hohlfaseranordnung ist quaderförmig ausgebildet.

Vorzugsweise handelt es sich bei der erfindungsgemäßen Vorrichtung um eine Vorrichtung ohne Wärmetauscher.

Mit der erfindungsgemäßen Vorrichtung ist es möglich, die Parameter Blutfluss, Priming-Volumen und Druckverlust sowohl der Blut- als auch der Gasseite und die Größe des Eingriffs zum Gefäßzugang bei hoher Effektivität der CO₂₋Eliminierung zu minimieren. Insbesondere wurde festgestellt, dass mit der erfindungsgemäßen Vorrichtung schon bei geringem Blutfluss von 500 ml pro Minute eine effektive CO₂-Eliminierung möglich ist. Aufgrund des geringen Blutflusses ist es möglich, mit kleinlumigen Kanülen oder sogar mit einer doppellumigen Kanüle zu arbeiten, die mit geringerer Invasivität beim Gefäßzugang, höherer Sicherheit durch bevorzugte venös-venös(W)-Kanülierung und damit verbundenem geringerem Blutdruckniveau und dadurch geringerem Überwachungsaufwand verbunden sind. Insbesondere ist es dadurch möglich, die erfindungsgemäße Vorrichtung nicht nur auf Intensivstationen zu verwenden. Das dem CO₂-Eliminator über einen Gaseinlass zugeführte Spülgas kann grundsätzlich beliebig gewählt werden. Die Anforderungen sind lediglich toxikologische Unbedenklichkeit und weitgehende Freiheit von CO₂. Insbesondere sind Luft oder Gemische von Luft mit Sauerstoff, Stickstoff oder Edelgasen geeignet, als Spülgas verwendet zu werden.

Alternativ oder zusätzlich kann vorgesehen sein, dass der Quotient aus aktiver Faserlänge und Gesamtfaserlänge < 0,79, vorzugsweise < 0,77, bevorzugt 0,724 ± 5% ist. Die aktive Faseroberfläche der Hohlfaseranordnung ist im Bereich 0,5 bis 1,2 m². Dabei weist die Hohlfaseranordnung mindestens 13.000, vorzugsweise mindestens 13.300 austauschaktive Fasern auf. Alternativ oder zusätzlich kann die Hohlfaseranordnung mindestens 13.300 Hohlfasern pro m² aktive Austauschoberfläche aufweisen. Mit einer solchen Ausgestaltung der Hohlfaseranordnung ist es möglich, einen annähernden Gleichgewichtszustand zwischen dem pCO₂ des Spülgases (nahe 0) und dem pCO₂ des behandelten Blutes zu erreichen. Dies bedeutet eine Abkehr von im Stand der Technik üblichen physiologischen Werten für pCO₂ (physiologischer Wert pCO₂ 32-46 mm Hg arteriell und 38-54 mm Hg venös) und pH-Wert (physiologischer Wert: pH 7,45-7,35 (arteriell-venös)).

Während es bei Anwendungen mit Oxygenatoren bekannt ist, einen hohen Gastransfer (ml transferiertes Gas pro Zeit) durch höhere Blutflüsse und geringere Änderungen der Blutpartialdrücke der betrachteten Gase vor bzw. nach dem Gasaustausch im Rahmen physiologischer Werte und hoher Differenzen zwischen den Partialdrucken der Gase und Austauschgase und dem Blut zu erreichen, wird durch die erfindungsgemäße Vorrichtung eine andere Vorgehensweise ermöglicht.

Die oben beschriebenen Gleichgewichtszustände sind mit bekannten Oxygenatoren nicht erreichbar, da diese optimiert wurden, um physiologisch normale Gaspartialdrücke aufrechtzuerhalten. Dabei wird über einen hohen Partialdruck von Sauerstoff im Versorgungsgas über den hohen vorliegenden Gradienten zwischen pO₂ in der Gasphase und im Blut eine hohe Transferleistung bei einem relativ hohen Blutfluss bewirkt, wenn ein physiologischer Sauerstoffpartialdruck gefordert wird. Würde das Blut mit diesem Versorgungsgas ins Gleichgewicht gebracht, würden extrem hohe und damit unphysiologische Sauerstoffpartialdrücke erzielt werden. Auch die Eliminierung von CO₂ bei der Blutoxygenierung soll bei bekannten Oxygenatoren zu physiologischen Konzentrationen am Ausgang des Oxygenators führen und nicht zu möglichst vollständiger Eliminierung von CO₂. Daher wird bei der Zielsetzung von physiologischen Verhältnissen im Stand der Technik mit hohem Gradienten der durch Behandlung zu verändernden physikalischen, chemischen oder biologischen Parameter und relativ kleiner und damit kostengünstiger Austauschfläche gearbeitet, die eine Gleichgewichtseinstellung nicht ermöglicht.

Versuche der Anmelderin haben überraschenderweise ergeben, dass bei geringen Blutflüssen die erfindungsgemäße Vorrichtung nahezu die gleiche Effektivität für die Entfernung von CO₂ aufweist wie ein an Format, Priming-Volumen und Austauschoberfläche deutlich größeres Gasaustauschmodul. Weiterhin wurde festgestellt, dass sehr hohe Gasflüsse die Annäherung an Gleichgewichtszustände (niedriger pCO₂ im Blut) fördern. Diese hohen Gasflüsse führen zu verhältnismäßig hohen Überdrücken, die bei Anwendungen von Oxygenatoren unüblich sind. Erreichen solche Überdrücke zwischen Gasseite und Blut den Bubble Point der Hohlfaseranordnung, ist der Übertritt von Gasbläschen ins Patientenblut möglich. Um solche kritischen Vorkommnisse zu vermeiden, sollte der Überdruck am Anfang der austauschaktiven Faser den Bubble Point nicht erreichen, bzw. der Überdruck auf der Gasseite sollte möglichst niedrig sein. Andererseits wurde festgestellt, dass größere Gasaustauschmodule selbst bei gleichem Gasfluss relativ zur vorhandenen Austauschfläche für die Entfernung von CO₂ uneffektiver waren als der erfindungsgemäße kleinformatige CO₂-Eliminator.

Die erfindungsgemäßen Vorteile können insbesondere erzielt werden, indem anders als bei Oxygenatoren sehr viele, kurze Hohlfasern verwendet werden.

Da durch den Verguss der Faserenden immer ein gewisser Prozentsatz der Hohlfaseranordnung für den Gasaustausch deaktiviert wird, wird im Stand der Technik versucht, diesen Prozentsatz durch Verwendung weniger, aber längerer Fasern gering zu halten.

Erfindungsgemäß bieten jedoch viele, kurze Fasern für die weitgehende Gleichgewichtseinstellung (eine Gleichgewichtseinstellung wird angestrebt, aber in dynamischen Systemen nie vollständig erreicht) zwischen dem pCO₂ des Spülgases (nahe 0) und dem pCO₂ des behandelten Blutes überraschenderweise mehrere Vorteile. Durch die vielen kurzen parallel geschalteten Hohlfasern reduziert sich der Strömungswiderstand und damit der Überdruck auf der Gasseite bei hohen Gasflüssen auf unkritische Werte. Damit werden sehr hohe Gasflüsse möglich, die die Effizienz der CO₂-Eliminierung fördern, ohne zu kritisch erhöhten Gasdrücken der Hohlfaseranordnung mit möglicher Gasblasenbildung im Blut zu führen. Das Spülgas erreicht während seiner Passage durch den kurzen austauschaktiven Teil der Hohlfaseranordnung durch Austausch nur geringe Werte von pCO₂, sodass im Verlauf der Passage ein höherer Gradient zwischen pCO₂ im Spülgas und im Blut erhalten bleibt. Eine längere Faser wäre durch einen höheren pCO₂ des Spülgases im weiteren Verlauf der Passage aufgrund des geringeren Gradienten uneffektiv.

Vorteilhafterweise kann zumindest ein Sensor zur Erfassung von Parametern des zu behandelnden Bluts vorgesehen sein. Insbesondere können die Sensoren in den CO₂-Elminator integriert sein. Anhand der erfassten Parameter kann die Effektivität der CO₂-Eliminierung überprüft werden. Ggf. kann der Gasfluss oder Blutfluss gesteuert oder geregelt werden.

Besonders vorteilhaft ist es daher, wenn eine Auswerteeinrichtung vorgesehen ist, die mit dem zumindest einen Sensor verbunden ist und mit einer Regeleinrichtung zur Regelung zumindest eines Parameters des extrakorporalen Kreislaufs in Verbindung steht.

Gemäß einer Ausgestaltung der Erfindung kann vorgesehen sein, dass mindestens zwei Kanülen (einlumige Kanülen) mit je einem Lumen < 21 French, bevorzugt mit je einem Lumen von 13 French, oder mindestens eine doppellumige Kanüle mit zwei Lumen < 24 French, bevorzugt mit Lumen von 19 French, vorgesehen sind. Durch diese Maßnahme kann die Invasivität vermindert werden.

Offenbart ist auch ein Verfahren zum Betrieb einer erfindungsgemäßen Vorrichtung, wobei ein Gleichgewicht zwischen dem pCO₂ des eintretenden Spülgases und dem pCO₂ des behandelten Bluts angestrebt wird. Dadurch kann eine sehr hohe Effektivität der CO₂-Eliminierung bei geringer Invasivität erreicht werden. Insbesondere können kleinlumige, insbesondere doppellumige veno-venöse Kanülen bei geringem Blutfluss eingesetzt werden. Durch die entsprechende Einstellung des Gleichgewichts lässt sich bei geringem Blutfluss eine weitgehende Befreiung des behandelten Bluts von CO₂ erreichen. Insbesondere kann ein pCO₂ des behandelten Bluts < 32 mm Hg, bevorzugt < 25 mm Hg, besonders bevorzugt < 15 mm Hg eingestellt werden, ein Wert, der selbst für arterielles Blut unphysiologisch ist und mit einem pH-Wert > 7,45, bevorzugt pH >7,6, besonders bevorzugt pH 7,8 einhergeht, was als respiratorische Alkalose bezeichnet wird.

In Versuchen der Anmelderin wurden beispielsweise pCO₂-Werte im Bereich von 10-15 mm Hg bei pH-Werten um etwa pH 7,8 im behandelten Blut ermittelt.

Bevorzugt ist es, wenn die Vorrichtung mit einem Blutfluss < 1200 ml pro Minute, besonders bevorzugt < 800 ml pro Minute, insbesondere < 500 ml pro Minute, betrieben wird. Dadurch senkt sich das Risiko für den behandelten Patienten. Außerdem können Kanülen und Leitungen mit geringerem Durchmesser verwendet werden.

Wie bereits erwähnt, kann vorteilhafterweise ein pH-Wert des behandelten Blutes > 7,45 eingestellt werden.

Hiermit wird auf die US-Patentanmeldung 61802335 vom 15.03.2013 Bezug genommen.

Weitere Merkmale und Vorteile der Erfindung ergeben sich aus der nachfolgenden Beschreibung eines Ausführungsbeispiels der Erfindung.

Es zeigen:
- Fig. 1: eine stark schematisierte Darstellung einer erfindungsgemäßen Vorrichtung;
- Fig. 2: eine Ansicht von oben auf einen CO₂-Eliminator;
- Fig. 3: eine Schnittdarstellung durch den CO₂-Eliminator gemäß der Linie A-A der Fig. 2;
- Fig. 4: eine Schnittdarstellung gemäß der Linie B-B der Fig. 3;
- Fig. 5: eine stark schematisierte Darstellung eines Ausschnitts einer Hohlfaseranordnung .

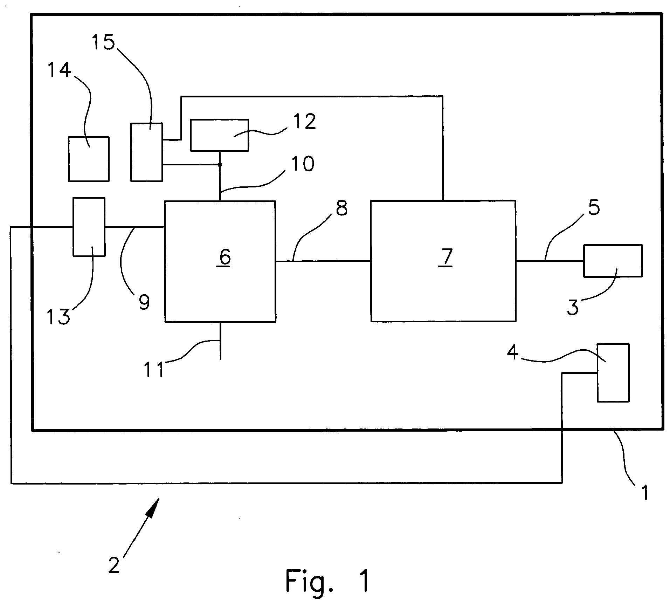

Die Fig. 1 zeigt eine Vorrichtung 1 in einem extrakorporalen Kreislauf 2. Die Vorrichtung 1 weist kleinlumige Kanülen 3, 4 zur Verbindung mit einem Patienten auf. Diese sind hier als einlumige Kanülen gezeigt, könnten jedoch als doppellumige Kanüle kombiniert sein. Weiterhin umfasst die Vorrichtung 1 ein Schlauchsystem 5. Zwischen der Kanüle 3 und einem CO₂-Eliminator 6 ist eine optionale Pumpe 7 vorgesehen. Die Pumpe kann beispielsweise als Rollerpumpe oder Zentrifugalpumpe ausgebildet sein. Bei der vorliegenden Anwendung kann eine okklusive Blutpumpe, z. B. eine Rollerpumpe, von Vorteil sein, da der Blutfluss weitgehend unabhängig von Druckverhältnissen proportional zu der Pumpendrehzahl ist. Somit kann sich eine Messung des Blutflusses erübrigen.

Der CO₂-Eliminator 6 weist einen Bluteinlass 8 und einen Blutauslass 9 auf. Weiterhin sind ein Gaseinlass 10 und ein Gasauslass 11 vorgesehen. Der Gaseinlass 10 ist an ein Spülgasreservoir 12, z. B. eine Gasleitung, ein Gasblender oder eine Gasflasche, angeschlossen.

Im Bereich des Blutauslasses 9 ist zumindest ein Sensor 13 angeordnet zur Erfassung von Parametern des behandelten Bluts. Der Sensor 13 ist mit einer Auswerteeinrichtung 14 verbunden, die wiederum mit einer Regeleinrichtung 15 verbunden ist. Durch die Regeleinrichtung 15 können in Abhängigkeit von den erfassten und ausgewerteten Parametern verschiedene Parameter des CO₂-Eliminierungsprozesses geregelt werden. Beispielsweise kann die Pumpe 7 geregelt werden oder der Gasdruck des Spülgases. Hierzu kann die Regeleinrichtung 15 beispielsweise mit einem Regelventil zwischen dem Spülgasreservoir 12 und dem Gaseinlass 10 verbunden sein.

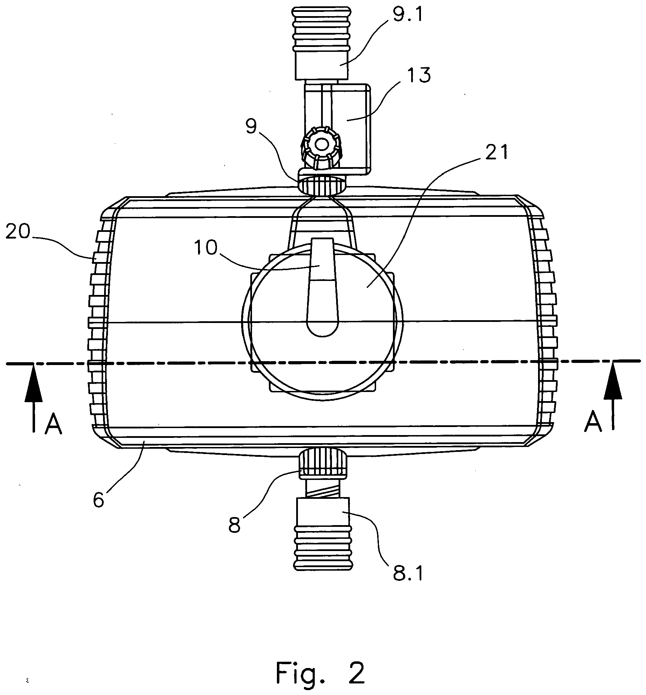

In der Fig. 2 ist der CO₂-Eliminator 6 in einer Draufsicht gezeigt. Hier ist zu erkennen, dass am Bluteinlass ein Konnektor 8.1 und am Blutauslass ein Konnektor 9.1 vorgesehen sind. Der Sensor 13 befindet sich dabei zwischen dem Blutauslass 9 und dem Konnektor 9.1. Der Sensor 13 ist beispielsweise eingerichtet, Temperatur, Blutfluss, Sauerstoffsättigung, pCO₂, pH oder andere Parameter des behandelten Bluts zu erfassen.

Der CO₂-Eliminator 6 weist ein Gehäuse 20 auf. Oben an dem Gehäuse ist ein Deckel 21 vorgesehen, an dem der Gaseinlass 10 angeordnet ist.

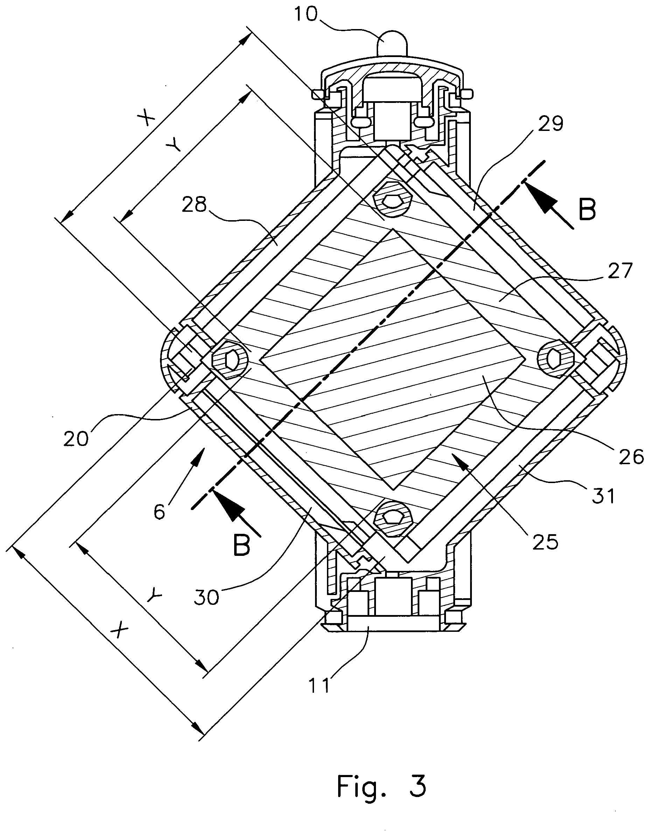

Die Fig. 3 zeigt eine Schnittdarstellung gemäß der Linie A-A der Fig. 2. Insbesondere ist hier zu erkennen, dass im Inneren des Gehäuses 20 des CO₂-Eliminators 6 eine Hohlfaseranordnung 25 vorgesehen ist, die mehrere Matten mit Hohlfasern aufweist. Dabei sind in einem austauschaktiven Bereich 26 unvergossene Fasern vorgesehen, während in einem Randbereich 27 die Fasern vergossen sind und somit nicht für den Gasaustausch aktiv sind. Die aktive Faserlänge ist dabei mit dem Buchstaben Y angegeben, d. h. einer Dimension des Querschnitts, der für den Gasaustausch aktiv ist. Die gesamte Faserlänge beträgt dagegen X und beinhaltet auch den Bereich 27, in dem die Fasern vergossen sind und somit nicht am Gasaustausch teilnehmen. Die Gesamtlänge der Fasern ist somit mit X und die austauschaktive Faserlänge bzw. aktive Faserlänge mit Y angegeben. In den Bereichen 28 befindet sich einströmendes Spülgas und in den Bereichen 30, 31 befindet sich Gas nach Durchströmen der Hohlfaseranordnung 25. Aus dieser Darstellung ergibt sich auch, dass aufgrund der Anordnung von Bluteinlass 8 und Blutauslass 9 der Blutfluss senkrecht zur Anordnung der Fasern der Hohlfaseranordnung 25 erfolgt.

Aus der Darstellung der Fig. 3 ergibt sich weiterhin, dass die Hohlfaseranordnung aus zwei (orthogonalen) Richtungen durchströmt wird, nämlich von den Bereichen 28, 29 aus.

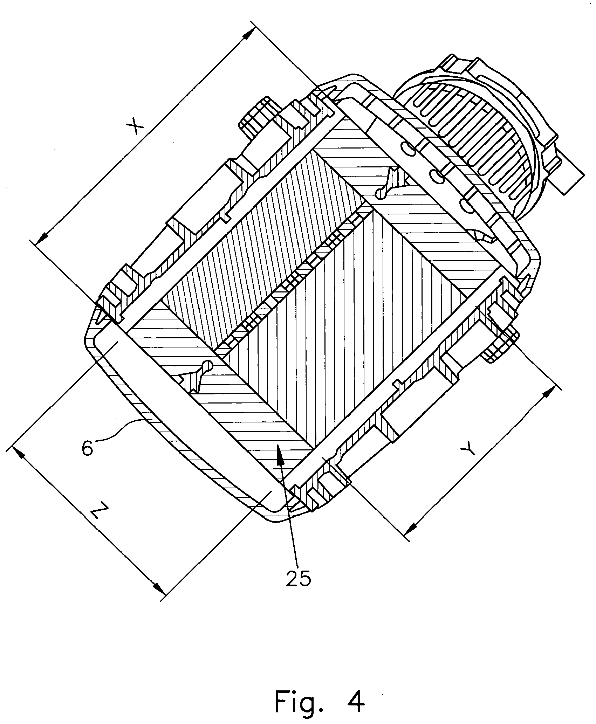

Die Fig. 4 zeigt eine Schnittdarstellung gemäß der Linie B-B der Fig. 3. Hier sind wiederum die aktive Faserlänge Y und die gesamte Faserlänge X eingezeichnet. Weiterhin ist hier die Dicke Z der Hohlfaseranordnung zu sehen. Die Dicke Z der Hohlfaseranordnung entspricht dabei der minimalen Wegstrecke der Blutpassage, d.h. dem kürzesten Weg, den das Blut durch die Hohlfaseranordnung nehmen kann. Der Blutfluss erfolgt hierbei im Wesentlichen rechtwinklig zur Ausrichtung der Hohlfasern in der Hohlfaseranordnung 25.

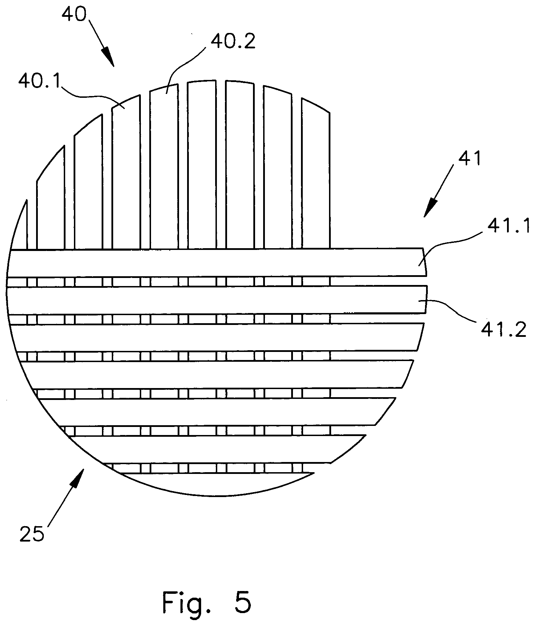

Die Fig. 5 zeigt eine erste (Hohlfaser-)Matte 40 mit mehreren Hohlfasern 40.1, 40.2, die parallel zueinander angeordnet sind. Weiterhin ist eine zweite Schicht oder Matte 41 mit Hohlfasern 41.1, 41.2 gezeigt, wobei auch die Hohlfasern 41.1, 41.2 parallel zueinander angeordnet sind. Die Hohlfasern 40.1, 40.2 sind jedoch rechtwinklig zu den Hohlfasern 41.1,41.2 angeordnet. Dies bedeutet, dass die Matten 40, 41 kreuzweise geschichtet sind.

Das von dem Spülgasreservoir 12 zugeführte Spülgas tritt über die eingangsseitigen Gasräume 28, 29 und Schnittflächen des Vergusses im Bereich 27 in die Hohlfasern 40.1, 40., 41.1, 41.2 ein, passiert deren Innenseite, wobei der austauschaktive Anteil Y der gesamten Faserlänge X den Gasaustausch bewirkt. Anschließend tritt das Gas an den Schnittflächen des Vergusses im Bereich 27 in die ausgangsseitigen Gasräume 30, 31 aus und entweicht über den an der Unterseite des CO₂-Eliminators 6 befindlichen Gasauslass 11.

Zur effektiven Eliminierung von CO₂ eines Blutflusses von etwa 500 ml pro Minute ist eine austauschaktive Oberfläche von etwa 0,5 m² bis 1,2 m² ausreichend. Die austauschaktiven Hohlfasern können entweder mikroporös oder diffusiv sein.

Besonders gute Ergebnisse wurden mit einem CO₂-Eliminator gemäß Ausführungsbeispiel 1 erzielt, dessen Hohlfaseranordnung eine aktive Fläche von 0,98 m² und mindestens 13 834 Hohlfasern bzw. mindestens 14116 Hohlfasern pro m² aufwies:

**Ausführungsbeispiel 1:**

| | |
|---|---|
| Oberfläche für Gasaustausch: | 0,98 m² |
| Maximale aktive Faserlänge (Y) : | 5,5 cm |
| Gesamtfaserlänge (X) : | 7,6 cm |
| Minimale Wegstrecke der Blutpassage (Z) | 5,4 cm |
| Minimale Faseranzahl | 13834 |
| Minimale Faseranzahl/m² | 14116 |

Daraus berechnet sich ein Quotient der maximalen aktiven Faserlänge (Y) und der minimalen Wegstrecke der Blutpassage (Z)
von Y/Z = 5,5 cm / 5,4 cm = 1,02.

Dieser kleine, nicht stark von 1 abweichende Quotient Y/Z ist das Kennzeichen eines sowohl blutseitig als auch gasseitig optimierten Strömungsweges und dadurch auch eines für beide Medien optimierten Strömungswiderstands.

Ein weiteres Merkmal der erfindungsgemäßen Vorrichtung ist der Quotient der maximalen aktiven Faserlänge (Y) und der Gesamtfaserlänge (X): Y/X= 5,5 cm/7,6 cm = 0,724. Das bedeutet, dass der austauschaktive Anteil der Fasern maximal 72,4% beträgt.

Die in DE 2011060U1 beschriebene Vorrichtung dagegen weist die folgenden Konstruktionsmerkmale auf:

| | |
|---|---|
| Oberfläche für Gasaustausch: | 1,3 m² |
| Minimale aktive Faserlänge Y: | 10,1 cm |
| Gesamtfaserlänge X : | 12,7 cm |
| Minimale Wegstrecke der Blutpassage Z | 2,6 cm |
| Maximale Faseranzahl | 11712 |
| Maximale Faseranzahl/m² | 9009 |

Daraus berechnet sich ein Quotient der aktiven Faserlänge (Y) und der minimalen Wegstrecke der Blutpassage (Z)
von Y/Z = 10,1 cm / 2,6 cm = 3,88.

Dieser deutlich höhere Quotient Y/Z zeigt an, dass in diesem Fall der geringe Strömungswiderstand des Blutes das Entwicklungsziel war.

Der Quotient der minimalen aktiven Faserlänge (Y) und der Gesamtfaserlänge (X) beträgt in diesem Fall:
Y/X = 10,1 cm/12,7cm = 0,795.

Das bedeutet, dass der austauschaktive Anteil der Fasern minimal 79,5% beträgt und damit deutlich höher ist, als der der erfindungsgemäßen Vorrichtung.

### Ausführungsbeispiel 2:

In einer weiteren Ausführung enthält das Gasaustauschmodul bei einer aktiven Fläche von 0,98 m² mindestens 13119 Hohlfasern bzw. mindestens 13300 Hohlfasern pro m²:

| | |
|---|---|
| Oberfläche für Gasaustausch: | 0,98 m² |
| Maximale aktive Faserlänge (Y) : | 5,8 cm |
| Gesamtfaserlänge (X) : | 7,6 cm |
| Minimale Wegstrecke der Blutpassage (Z) | 5,4 cm |
| Minimale Faseranzahl | 13119 |
| Minimale Faseranzahl/m² | 13300 |

Daraus berechnet sich ein Quotient der maximalen aktiven Faserlänge (Y) und der minimalen Wegstrecke der Blutpassage (Z)
von Y/Z = 5,8 cm / 5,4 cm = 1,07.

Dieser kleine, nicht stark von 1 abweichende Quotient Y/Z ist das Kennzeichen eines sowohl blutseitig als auch gasseitig optimierten Strömungsweges und dadurch auch eines für beide Medien optimierten Strömungswiderstands.

Der Quotient der maximalen aktiven Faserlänge (Y) und der Gesamtfaserlänge (X) beträgt:
Y/X = 5,8 cm/7,6 cm = 0,763.
Das bedeutet, dass der austauschaktive Anteil der Fasern maximal 76,3% beträgt.

### Ausführungsbeispiel 3:

In einer weiteren Ausführung enthält das Gasaustauschmodul bei einer aktiven Fläche von 0,98 m² und Verwendung von Hohlfasern an der unteren Grenze der Spezifikation (Außendurchmesser 0,35 mm) mindestens 17 148 Hohlfasern bzw. mindestens 17 497 Hohlfasern pro m²:

| | |
|---|---|
| Oberfläche für Gasaustausch: | 0,98 m² |
| Maximale aktive Faserlänge (Y) : | 5,2 cm |
| Gesamtfaserlänge (X) : | 7,6 cm |
| Minimale Wegstrecke der Blutpassage (Z) | 5,4 cm |
| Minimale Faseranzahl | 17148 |
| Minimale Faseranzahl/m² | 17497 |

Daraus berechnet sich ein Quotient der maximalen aktiven Faserlänge (Y) und der minimalen Wegstrecke der Blutpassage (Z)
von Y/Z = 5,2 cm / 5,4 cm = 0,963.

Dieser kleine, nicht stark von 1 abweichende Quotient Y/Z ist das Kennzeichen eines sowohl blutseitig als auch gasseitig optimierten Strömungsweges und dadurch auch eines für beide Medien optimierten Strömungswiderstands.

Der Quotient der maximalen aktiven Faserlänge (Y) und der Gesamtfaserlänge (X) beträgt:
Y/X = 5,2 cm/7,6 cm = 0,684.

## Patentansprüche

1. Vorrichtung (1) zur zumindest teilweisen CO₂ -Eliminierung von Patientenblut im extrakorporalen Kreislauf (2) mit einem CO₂-Eliminator (6), der ein Gehäuse (20) mit einem Bluteinlass (8) und einem Blutauslass (9) sowie einem Gaseinlass (10) und einem Gasauslass (11) aufweist, wobei im Gehäuse (20) eine sowohl von Gas als auch Blut durchströmbare Hohlfaseranordnung (25) mit einer für den Gasaustausch aktiven Faserlänge (Y) als Anteil einer Gesamtfaserlänge (X) vorgesehen ist, wobei die Hohlfasern in Matten angeordnet sind, wobei die Fasern in den Matten parallel ausgerichtet sind und die Matten in der Hohlfaseranordnung gestapelt sind, sodass die Fasern in parallelen benachbarten Matten in etwa rechtwinklig zueinander stehen, und dass die Wegstrecke der Blutpassage, die der Dicke (Z) der Hohlfaseranordnung (25) entspricht, im Wesentlichen rechtwinklig zur Faserorientierung und auf kürzestem Wege durch die Hohlfaseranordnung (25) verläuft, wobei die Hohlfaseranordnung (25) quaderförmig ausgebildet ist und der Quotient aus aktiver Faserlänge (Y) und minimaler Wegstrecke (Z) der Blutpassage durch die Hohlfaseranordnung (25) kleiner 3,5, vorzugsweise kleiner 3, besonders bevorzugt kleiner 2, ganz besonders bevorzugt kleiner 1,1 ist, wobei in einem austauschaktiven Bereich (26) unvergossene Fasern vorgesehen, während in einem Randbereich (27) die Fasern vergossen sind und somit nicht für den Gasaustausch aktiv sind, wobei die Hohlfaseranordnung (25) einen quadratischen Querschnitt aufweist, dessen Seitenlänge der Gesamtfaserlänge (X) entspricht und der austauschaktive Bereich (26) einen quadratischen Querschnitt aufweist, dessen Seitenlänge der aktiven Faserlänge (Y) entspricht, **dadurch gekennzeichnet, dass** die für den Gasaustausch aktive Faseroberfläche der Hohlfaseranordnung im Bereich 0,5 - 1,2 m² liegt.

2. Vorrichtung nach Anspruch 1, **dadurch gekennzeichnet, dass** der Quotient aus aktiver Faserlänge (Y) und Gesamtfaserlänge (X) kleiner 0,79, vorzugsweise kleiner 0,77, bevorzugt 0,724 +/- 5% ist.

3. Vorrichtung nach einem der vorhergehenden Ansprüche, **dadurch gekennzeichnet, dass** die Hohlfaseranordnung (25) mindestens 13300 Hohlfasern (40.1, 40.2, 41.1, 41.2) pro m² aktive Austauschoberfläche aufweist.

4. Vorrichtung nach einem der vorhergehenden Ansprüche, **dadurch gekennzeichnet, dass** zumindest ein Sensor (13) zur Erfassung von Parametern des behandelten Bluts vorgesehen ist.

5. Vorrichtung nach Anspruch 4, **dadurch gekennzeichnet, dass** eine Auswerteeinrichtung(14) vorgesehen ist, die mit dem zumindest einen Sensor (13) verbunden ist und mit einer Regeleinrichtung (15) zur Regelung zumindest eines Parameters des extrakorporalen Kreislaufs (2) in Verbindung steht.

6. Vorrichtung nach einem der vorhergehenden Ansprüche, **dadurch gekennzeichnet, dass** Kanülen (3, 4) mit einem Lumen kleiner als 21 French, bevorzugt von 13 French, oder doppellumige Kanülen (3, 4) mit einem Lumen kleiner als 24 French, bevorzugt 19 French, vorgesehen sind.

7. Vorrichtung nach einem der vorhergehenden Ansprüche, **dadurch gekennzeichnet, dass** sie keinen Wärmetauscher aufweist.

## Claims

1. A device (1) for at least partial CO₂ elimination from patient blood in an extracorporeal circuit (2), having a CO₂ eliminator (6) which comprises a housing (20) with a blood inlet (8) and a blood outlet (9) as well as a gas inlet (10) and a gas outlet (11), wherein a hollow fiber arrangement (25) is provided in the housing (20), through which arrangement both gas and blood can flow and which has an active fiber length (Y) for gas exchange as part of an overall fiber length (X), wherein the hollow fibers are arranged in mats, wherein the fibers in the mats are aligned in parallel and the mats are stacked in the hollow fiber arrangement, such that fibers in parallel adjacent mats are approximately at a right angle to each other, and that the length of the passageway for blood, which corresponds to the thickness (Z) of the hollow fiber arrangement (25), is substantially at a right angle to the fiber orientation and extends along the shortest path through the hollow fiber arrangement (25), wherein the hollow fiber arrangement (25) has a cuboid shape and the quotient of active fiber length (Y) and minimum length (Z) of the passageway for blood through the hollow fiber arrangement (25) is less than 3.5, preferably less than 3, particularly preferably less than 2, most preferably less than 1.1, wherein the fibers provided in the active exchange region (26) are unsealed while the fibers in a peripheral region (27) are sealed and thus not active for gas exchange, wherein the hollow fiber arrangement (25) has a square cross section whose side length corresponds to the overall fiber length (X) and the active exchange region (26) has a square cross section whose side length corresponds to the active fiber length (Y), **characterized in that** the fiber surface area of the hollow fiber arrangement which is active for gas exchange is in the range from 0.5 to 1.2 m².

2. The device according to claim 1, **characterized in that** the quotient of active fiber length (Y) and overall fiber length (X) is less than 0.79, preferably less than 0.77, particularly preferably 0.724 +/- 5%.

3. The device according to any one of the preceding claims, **characterized in that** the hollow fiber arrangement (25) comprises at least 13300 hollow fibers (40.1, 40.2, 41.1, 41.2) per m² of active exchange surface area.

4. The device according to any one of the preceding claims, **characterized in that** at least one sensor (13) is provided for detecting parameters of the treated blood.

5. The device according to claim 4, **characterized in that** an evaluation device (14) is provided, which is in communication with the at least one sensor (13) and with a control device (15) for controlling at least one parameter of the extracorporeal circuit (2).

6. The device according to any one of the preceding claims, **characterized in that** needles (3, 4) are provided having a lumen of less than 21 French, preferably 13 French, or double lumen needles (3, 4) are provided having a lumen of less than 24 French, preferably 19 French.

7. The device according to any one of the preceding claims, **characterized in that** it does not comprise a heat exchanger.

## Revendications

1. Dispositif (1) d'élimination au moins partielle de CO₂ du sang d'un patient dans la circulation extracorporelle (2) avec un éliminateur de CO₂ (6) qui présente un boîtier (20) avec une entrée de sang (8) et une sortie de sang (9) ainsi qu'une entrée de gaz (10) et une sortie de gaz (11), dans lequel dans le boîtier (20) un agencement de fibres creuses (25) avec une longueur de fibre (Y) active pour l'échange gazeux en tant que partie d'une longueur de fibre totale (X), pouvant être traversé aussi bien par du gaz que par du sang est prévu, dans lequel les fibres creuses sont agencées en tapis, dans lequel les fibres des tapis sont orientées parallèlement et les tapis dans l'agencement de fibres creuses sont empilées, de sorte que les fibres dans des tapis parallèles voisins se trouvent à peu près angle droit les unes par rapport aux autres, et en ce que le trajet du passage de sang qui correspond à l'épaisseur (Z) de l'agencement de fibres creuses (25) évolue sensiblement à angle droit par rapport à l'orientation des fibres et évolue sur le chemin le plus court, au travers de l'agencement de fibres creuses (25), dans lequel l'agencement de fibres creuses (25) est conçu de façon parallélépipédique et le quotient de longueur de fibre active (Y) et de trajet minimal (Z) du passage de sang au travers de l'agencement de fibres creuses (25) est inférieur à 3,5, de préférence inférieur à 3, de manière particulièrement préférée, inférieur à 2, de manière tout particulièrement préférée inférieur à 1,1, dans lequel dans une zone d'échange actif (26) des fibres non encapsulées sont prévues, tandis que dans une zone périphérique (27) les fibres sont encapsulées et ainsi ne sont pas actives pour l'échange gazeux, dans lequel l'agencement de fibres creuses (25) présente une section carrée dont la longueur latérale correspond à la longueur totale de fibre (X) et la zone d'échange actif (26) présente une section carrée dont la longueur latérale correspond à la longueur de fibre active (Y), **caractérisé en ce que** la surface de fibre active pour l'échange gazeux de l'agencement de fibres creuses se situe dans la plage de 0,5 à 1,2 m².

2. Dispositif selon la revendication 1, **caractérisé en ce que** le quotient de longueur de fibre active (Y) et de longueur totale de fibre (X) est inférieur à 0,79, de préférence inférieur à 0,77, de préférence de 0,724 +/- 5 %.

3. Dispositif selon l'une quelconque des revendications précédentes, **caractérisé en ce que** l'agencement de fibres creuses (25) présente au moins 13 300 fibres creuses (40,1, 40,2, 41,1, 41,2) par m² de surface d'échange actif.

4. Dispositif selon l'une quelconque des revendications précédentes, **caractérisé en ce qu'**au moins un capteur (13) est prévu pour la saisie de paramètres du sang traité.

5. Dispositif selon la revendication 4, **caractérisé en ce que** qu'un système d'analyse (14) est prévu, qui est relié à au moins un capteur (13) et est en communication avec un système de réglage (15) pour le réglage d'au moins un paramètre de la circulation extracorporelle (2).

6. Dispositif selon l'une quelconque des revendications précédentes, **caractérisé en ce que** des canules (3, 4) présentant une lumière inférieure à 21 French, de préférence de 13 French, ou des canules à double lumière (3, 4) présentant une lumière inférieure à 24 French, de préférence 19 French, sont prévues.

7. Dispositif selon l'une quelconque des revendications précédentes, **caractérisé en ce qu'**il ne présente pas d'échangeur de chaleur.
